# EUROPEAN PATENT APPLICATION

(11) **EP 0 982 317 A1**
(43) Date of publication of application: **01.03.2000**
(21) Application number: 98116127.6
(22) Date of filing: 26.08.1998
(51) Int. Cl.: C07K 5/062, A61K 47/48, A61K 38/05

(54) **Bivalent inhibitors of the proteasome**

(71) Applicant: Max-Planck-Gesellschaft zur Förderung der Wissenschaften e.V., 80539 München (DE)
(72) Inventor: Ditzel, Lars, 36142 Tann 1, a.d. Rhön (DE); Groll, Michael, 94072 Bad Füssing (DE); Huber, Robert, Prof. Dr., 82110 Germering (DE); Loidl, Günther, 93333 Neustadt/Donau (DE); Musiol, Hans-Jürgen, 81475 München (DE); Moroder, Luis, Prof. Dr., 82152 Martinsried (DE)
(74) Representative: Huber, Bernhard, Dipl.-Chem.

(57) **Abstract**

A bivalent proteasome inhibitor of the general formula I

X-CH(R1)-NH-[P]ₙ-Y-Z-Y'-[P']ₘ-NH-CH(R1')-X'

wherein X and X' are identical or different and are groups selected from aldehyde, boronic acid, boronic acid ester, ketoaldehyde, ketoester, ketoamide, chloromethylketone, trifluoromethylketone, diazomethane;
R1 and R1' are identical or different and correspond to the specificity of the S1 enzyme subsite for chymotrypsin- or trypsin-like or PGPH activity of the proteasome, selected from hydrophobic side chains of L- or D-natural and -unnatural amino acids, or amino-, guanido-, amidino-alkyl- or cycloalkyl-, alkaryl-, aralkyl residues and carboxy-alkyl- or aryl- or alkaryl- or aralkyl residues;
P and P' are identical or different and correspond to the P2 to P7 substrate positions and are natural or unnatural L- or D-amino acids with hydrophobic or hydrophylic or charged side chains;
m and n are identical or different and are 0, 1, 2, 3, 4, 5 or 6;
Y and Y' are identical or different and correspond to the formula CO-(A)-(B) where (A) is an alkyl, alkenyl, cycloalkyl, alkaryl, aralkyl or heteroaromatic group and (B) is CO or NH or omitted;
and Z is O-CH₂-CH₂-(O-CH₂-CH₂)ₙ-O, OC-CH₂-(O-CH₂-CH₂)ₙ₋₁-O-CH₂-CO or NH-CH₂-CH₂-(O-CH₂-CH₂)ₙ-NH with n varying from 4 to 50 has interesting properties for various drug applications.

## Description

The invention comprises new proteasome inhibitors that are based on the use of polyethyleneglycol (PEG) as a flexible and linear spacer of defined length to crosslink two head groups capable of interacting with the proteases-like active sites of the multienzyme complex and thus to produce bivalent inhibitors of high affinity and selectivity.

The proteasome is a multicatalytic protease complex that is responsible for the intracellular protein turnover in procaryotes and eucaryotes. As it is involved in many pathophysiological processes like inflammation and cancer, it constitutes a promising target for drug design.

The main biological functions of the proteasome in both the cytosol and the nucleus are removal of abnormal, misfolded or improperly assembled proteins, stress response (by processing or degradation of transcriptional regulators), cell cycle control (by degradation of cyclins), cell differentiation and metabolic adaptation (by destruction of transcription factors or metabolic enzymes), and the cellular immune response (by generating antigenic peptides presented by MHC class I molecules). These cellular functions are linked to an ubiquitin and ATP requiring protein degradation pathway involving the 26S proteasome whose core and proteolytic chamber is formed by the 20S proteasome. Its essential nature is reflected in the lethality of chromosomal deletions of all but one (a3/Y13)' of its subunits in yeast.^{2,2a,3,4,5} Proteasomes are ubiquitous in all three kingdoms of life.

The X-ray structure of 20S proteasome from the archaebacterium *Thermoplasma acidophilum* at 3.4 Å resolution showed that it is a particle of cylindrical shape in which the 28 subunits are arranged as four homo-heptameric rings α₇β₇β₇α₇.⁶Since the N-terminal threonine residue of the β-subunits was found to bind via hemiacetal formation to inhibitory peptide aldehydes, this residue is involved in the hydrolytic activity,^{6,7} relating proteasome to the Ntr ('N-terminal nucleophile') family of hydrolases.⁸

The yeast *Saccharomyces cerevisiae* 20S proteasome is similar in size and shape to the archaebacterial complex, but it is much more complex with 7 different α-type and 7 different β-type subunits, all of which have been cloned and sequenced and can be grouped by sequence homology.⁹ As defined by the character of the P1 cleavage sites of chromogenic substrates, trypsin-like, chymotrypsin-like, and peptidylglutarnyl-peptide hydrolytic (PGPH) activities are exhibited by the eukaryotic proteasome. These specificities, however, are not reflected in the cleavage pattern of protein substrates, which are instead cleaved at almost every position.¹⁰⁻¹³

The crystal structure of the 20S proteasome from the yeast *Saccharomyces cerevisiae* showed that its 28 protein subunits are arranged as an (α1.....α7, β1...β7)₂ complex in four stacked rings.¹⁴ The active sites are again located in the interior of the particle which is apparently accessible only by some very narrow side entries. Three of the seven different β-type subunits are processed at the threonine-1 residue and display inhibitor binding sites suggesting that the β5/PRE2 subunit has both a chymotrypsin-like and a trypsin-like specificity and that the β1/PRE3 has PGPH specificity. The β2/PUP1 can accomodate very large P1 residues of neutral, acidic or basic character.

The eukaryotic 20S proteasomes, including those from yeast, are very closely related in function, amino acid sequences of their components, and in the structure as shown by electronmicroscopy. The α-type and β-type subunits of the mammalian 20S proteasome are arranged in an ordered and unique way.¹⁵ In mammalian cells, three additional non-essential subunits of the 20S proteasome LMP2, LMP7, and MECL1 can replace constitutive components upon induction by the T-cell derived antiviral cytokine interferon-γ. The LMP subunits are encoded in a region of the MHC gene cluster adjacent to the TAP (transporters associated with antigen-presentation ) genes, which are involved in transport of antigenic peptides from the cytosol to the endoplasmic reticulum. Their expression or targeted deletion alters the proteasome's peptidase specificity and MHC class I cell-surface expression level.¹⁶⁻¹⁹ The properties of peptide fragments generated by the proteasome correlate strongly with those of MHC class I ligands and processing by proteasomes is probably the major pathway of MHC class I peptide generation.⁴

Once the proteasome was thought to be responsible only for the catabolism of abnormal proteins, now it is known that the degradation pathway by the proteasome plays a key role in the turnover and processing of many regulatory proteins.² Several of these proteolytic processes represent key biochemical events that contribute to the pathogenesis of human diseases. Thus , the proteasome is now being recognized as an important molecular target for drug discovery by developing specific and potent inhibitors.²⁰

The Ntr character of the active sites of the proteasome is very similar to the active sites of serine and cysteine proteases and thus, the knowledge accumulated in the past for developing reversible and irreversible inhibitors of serine and cysteine proteases has been applied for the design of proteasome inhibitors.

Among the irreversible inhibitors reported for the proteasome like 3,4-dichloroisocoumarin,^{21,22} peptidyl-α'β'-epoxyketones,²³ peptidyl-vinylsulfones,^{24,25} peptidyl-chloromethyl and -diazomethyl-ketones²⁶ and lactacystine,²⁷ only lactacystine shows a high degree of selectivity. Since irreversible inhibitors are of limited therapeutical interest, great attention has been paid in recent years to the design and synthesis of reversible inhibitors by focusing mainly on the use of peptide aldehydes, peptide boronic acids and their esters (for review see ref. 20), peptide ketoaldehydes²⁸ and ketocarbonyl derivatives.²⁹ The binding mode of the peptide aldehydes is based on hemiacetal formation between the Thr1Oγ of the active site and the carbonyl carbon of the aldehyde as well assessed by X-ray analysis of proteasome/peptide aldehyde adducts.^{6,14,30,31} Independently of the C-terminal anchor group mainly large size hydrophobic side chains have been exploited in position P1, P2 and P3 to develop highly potent inhibitors of the chymotryptic activity of the proteasome as well described in WO 95/25533 and ref. 30. Thereby cross-inhibition of cathespin B and calpain represents a major obstacle for therapeutical applications.

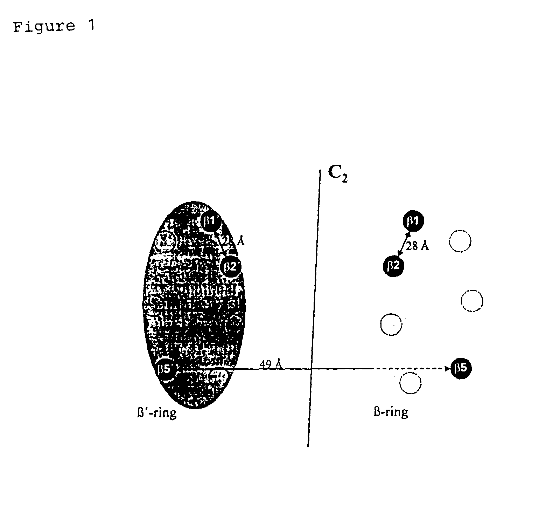

The crystal structure of the 20S proteasome from *Saccharomyces cerevisiae*¹⁴ revealed a symmetrical arrangement of the three active sites with their PGPH, trypsin- and chymotrypsin-like specifities at the level of each of the two central β-rings in the cavity of the cylindrical particle as schematically outlined in Figure 1, providing a schematic presentation of the active sites and their intra- and inter-ring distances: β1 and β1' (PGPH), β2 and β2' (trpysin-linke activity), β5 and β5' (chymotrypsin-like activity). Well defined intra- and inter-ring distances between the different active sites can be derived from this crystal structure.

The present invention describes the design and synthesis of bivalent inhibitors based on these specific distances. Thermodynamically the binding of multivalent molecules to different binding sites is similar to the chelate effect.³²⁻³⁴ The common feature of these binding events is that following the initial recognition and binding or reaction at one active site, each succeeding event, i.e. recognition and binding at a second active site, is more favorable because the entropy loss is decreased. Thus, exploiting these beneficial entropic effects of multivalency on avidity, we expect that binding affinities can be increased by ordcrs of magnitude, if an inhibitor would be available containing two active site binding moities spaced apart by a properly designed molecule.

**Table 1.**

| The Oγ→Oγ distances between two Thr-1 residues of the different active sites of the proteasome from *S. cerevisiae* as derived from the coordinates of the X-ray structure.¹⁴ | | | |
|---|---|---|---|
| **intra-ring distances** | | **inter-ring distances** | |
| β1 β2 | 27,95 Å | β1 β1' | 29,16 Å |
| β2 β5 | 64,94 Å | β2 β2' | 64,99 Å |
| β1 β2 | 64,38 Å | β5 β5' | 49,44 Å |
| | | β1 β2' | 50,45 Å |
| | | β1 β5' | 60,60 Å |
| | | β2 β5' | 42,86 Å |

For the design of the bivalent inhibitors capable of spanning distances of 50 Å or more, the main concern is the choice of the spacer. Taking into account the ubiquitinproteasome pathway of protein degradation and the information derived from the X-ray structure of the proteasome, for a protein to be hydrolyzed by the proteasome, it must first be completely unwinded and cystine bridges must be reduced and the linear polypeptide chain is then threaded through a narrow passage into the inner channel of the proteasome for proteolysis. Correspondingly, bulky scaffolds can not be used for oligo-presentation of the inhibitors. Since the spacer should mimick as much as possible the unstructured polypeptide chain of an unfolded protein, peptides of the aproppriate length would be best suited for such purpose. However, peptides of this size like gastrin (17mer) or secretin (27mer) were found to be rapidly degraded, fully confirming previous studies on the degradation of peptides by the proteasome.¹² Additionally, the spacer should be highly hydrophilic in order to avoid hydrophobic collapses that would prevent the molecule to be threaded into the cylinder in an extended unfolded form.

Therefore it is an object of the invention to develop a bivalent inhibitor of the proteasome containing a spacer possessing the requested properties that is not degraded by proteolysis and bearing two monovalent inhibitor groups spaced by at least about 50 Å. We found the right spacer with polyoxyethylene (PEG). PEG is a fully water-soluble and fully unstructured linear polymer that mimics perfectly unstructured polypeptide chains. Therefore the problem defined above was solved according to the invention by providing bivalent inhibitors of the general formula I containing PEG as spacer:

**X-CH(R1)-NH-[P]**_{**n**}**-Y-Z-Y'-[P']**_{**m**}**-NH-CH(R1')-X'** (general formula I)

wherein X and X' are identical or different and are groups selected from aldehyde, boronic acid, boronic acid ester, ketoaldehyde, ketoester, ketoamide, chloromethylketone, trifluoromethylketone, diazomethane;

R1 and R1' are identical or different and correspond to the specificity of the S1 enzyme subsite for chymotrypsin- or trypsin-like or PGPH activity of the proteasome, selected from hydrophobic side chains of L- or D-natural and -unnatural amino acids or amino-, guanido-, amidino-alkyl- or cycloalkyl-, alkaryl-, aralkyl and residues carboxy-alkyl- or aryl- or alkaryl- or -aralkyl residues;

P and P' are identical or different and correspond to the P2 to P7 substrate positions and are natural or unnatural L- or D-amino acids with hydrophobic or hydrophilic or charged side chains;

m and n are identical or different and are 0, 1, 2, 3, 4, 5 or 6;

Y and Y' are identical or different and correspond to the formula CO-(A)-(B) where (A) is an alkyl, alkenyl, cycloalkyl, alkaryl, aralkyl or heteroaromatic group and (B) is CO or NH or omitted;

and Z is O-CH₂-CH₂-(O-CH₂-CH₂)ₙ-O, OC-CH₂-(O-CH₂-CH₂)ₙ₋₁-O-CH₂-CO or NH-CH₂-CH₂-(O-CH₂-CH₂)ₙ-NH with n varying from 4 to 50.

Preferred hydrophobic side chains within the definition of R1 and R1' are linear or branched alkyl, cycloalkyl, alkenyl, aryl and -CH₂R2 where R2 is aryl, aralkyl, alkaryl, cycloalkyl or -Q-R3 where Q is a chalcogen, and R3 is alkyl.

In the general formula I alkyl and and alkenyl residues may contain from 1 to 20 resp. 2 to 20 C-atoms, preferably 1 to 12 resp. 2 to 12 C-atoms, especially 1 to 8 resp. 2 to 8 C-atoms if bound to an aryl residue.

The inhibition potency of a compound of the general formula I for the chymotryptic activity of proteasome in comparison with the related monovalent inhibitor (**1**) is shown as example in Table 2. As the active sites with chymotryptic activity are located at a distance of 50 Å and the inhibitor moiety binds to the S sites of the active center, commercial diamino-PEG with a statistical length distribution of 18 to 25 monomers and with the terminal amino groups capped with succinic acid was used to crosslink two molecules of H-Leu-Leu-Nle-H for the production of the bis-aldehyde **3**. To analyze the effect of the PEG moiety on binding affinities the monoaldehyde **2** was prepared. As shown in Table 1 the bivalent inhibitor **3** corresponding to the invention showed an increase in binding affinity of about two orders of magnitude if compared to inhibitor **1** or to the monovalent inhibitor **2**.

**Table 1.**

| *Inhibition of yeast proteasome by mono- and bivalent inhibitors (IC*^{*50*}*, µM).* | | | |
|---|---|---|---|
| **Inhibitor** | β1/β1' | β2/β2' | β5/β5' |
| Ac-Leu-Leu-Nle-H (**1**) | >100 | >100 | 2.1 |
| HOOC-(CH₂)₂-CO-NH-(PEG)₁₈₋₂₅-NH-(CH₂)₂-CO-Leu-Leu-Nle-H(**2**) | >100 | >100 | 1.8 |
| (PEG)₁₈₋₂₅-[NH-CO-(CH₂)₂-CO-Leu-Leu-Nle-H]₂ (**3**) | >100 | >100 | 0.017 |

In the case of bivalent inhibitors as head groups inhibitory moieties that show *per se* weak affinity can be used and enhancement of affinity is obtained by the bivalency. Thus, moieties which as monovalent inhibitors are poorly inhibiting other serine or cysteine proteases such as calpain or cathepsin B can be used to obtain potent proteasome inhibitors of correspondingly high specificity.

Therefore in another embodiment of the invention the potency of monovalent proteasome inhibitors is markedly increased by linking two such inhibitors together by a PEG-spacer group spanning a distance of at least about 50 Å. Preferred monovalent inhibitors are the reactive handles known form the state of art of serine and cysteine protease inhibitors.

The following examples describe embodiments of the invention further.

### Example 1

### Proteasome assay

A solution of proteasome from *Saccharomyces cerivisiae* in Tris-buffer (pH 7.5; 450 µl; 6.67 nM for PGPH, 5.56 nM for trypsin-like and 1.11 nM for chymotrypsin-like activity) was incubated with the inhibitors at varying concentrations (1 mnol to 100 µmol) for 1 h at 37°C. The fluorogenic substrates for PGPH (Z-Leu-Leu-Gly-βNA, 40 µM), trypsin-like (Bz-Phe-Val-Arg-AMC, 8 µM) and chymotrypsin-like assays (Suc-Leu-Leu-Val-Tyr-AMC, 8 µM) were dissolved in the same Tris-buffer with a minimum amount of DMSO and added to the enzyme solution at 37 °C to reach a final volume of 500 µl. Fluorescence excitation/emission wavelengths were 360 nm/460 nm for AMC and 335 nm/410 nm for βNA. The rates of hydrolysis were monitored by the fluorescence increase and the initial linear portions of curves (100-300 sec) were used to calculate the IC₅₀ values.

### Example 2

### Inhibitor-Synthesis

### H-Leu-Leu-Nle-Sc trifluoroacetate

The title compound was prepared as described previously.³⁵

### Ac-Leu-Leu-Nle-H (1)

The compound was obtained by acetylation of H-Leu-Leu-Nle-Sc trifluoroacetate with acetic anhydride followed by hydrolysis of the semicarbazone derivative.³⁰

### HOOC-(CH₂)₂-CO-NH-(PEG)₁₈₋₂₅-NH-(CH₂)₂-CO-Leu-Leu-Nle-Sc

The succinyl-amino-(PEG)₁₈₋₂₅ (Rapp Polymere, Tübingen, Germany) (100 mg, 88 µmol), Leu-Leu-Nle-Sc trifluoroacetate³⁵ (45 mg, 88 µmol), TBTU (28 mg, 88 µmol), HOBt (12 mg, 88 µmol) and DIEA (45 µl, 264 µmol) were dissolved in 60 ml DMF. After 3 h the solvent was evaporated and the oily residue was distributed between methylene chloride and 5% KHSO₄. The aqueous phase was extracted twice with methylene chloride and the combined organic layers were washed with 5% NaHCO₃, dried over Na₂SO₄ and evaporated *in vacuo* to a colorless oil; yield: 90 mg (68%); HPLC [column: Nucleosil 300/C8 (Macherey & Nagel, Düren, Germany), linear gradient of acetonitrile/2% phosphoric acid from 5:95 to 80:20 in 30 min at a flow rate of 1 ml/min]: Rₜ = 7.70 min.

### (PEG)₁₈₋₂₅-[NH-CO-(CH₂)₂-CO-Leu-Leu-Nle-Sc]₂

The succinyl-amino-(PEG)₁₈₋₂₅ (Rapp Polymere, Tübingen, Germany) (100 mg, 88µmol), Leu-Leu-Nle-Sc trifluoroacetate³⁶ (90 mg, 176 µmol), TBTU (57 mg, 176 µmol), HOBt (24 mg, 176 µmol) and DIEA (90 µl, 528 µmol) were dissolved in 1 ml DMF. After 4h the solvent was evaporated and the oily residue was purified by preparative HPLC on Nucleosil 250/C18 (Macherey & Nagel, Düren, Germany) with a linear gradient of acetonitrile/water from 15:85 to 60:40 in 70 min. Fractions containing homogeneous material as monitored by HPLC were combined and lyophilized; yield: 80 mg of a colorless oil (49%); HPLC [column: Nucleosil 300/C8 (Macherey & Nagel, Düren, Germany), linear gradient of acetonitrile/2% phosphoric acid from 5:95 to 80:20 in 30 min at a flow rate of 1 ml/min]: Rₜ = 8.73 min.

### HOOC-(CH₂)₂-CO-NH-(PEG)₁₈₋₂₅-NH-(CH₂)₂-CO-Leu-Leu-Nle-H (2)

HOOC-(CH₂)₂-CO-NH-(PEG)₁₈₋₂₅-NH-(CH₂)₂-CO-Leu-Leu-Nle-Sc (80 mg, 53 µmol) was dissolved in a mixture of 5 ml MeOH, 2 ml acetic acid and 2 ml formaldehyde (37 % in water). After 2 h the solvent was removed and the oily residue was immediately purified by preparative HPLC on Nucleosil 250/C 18 (Macherey & Nagel, Düren, Germany) with a linear gradient of acetonitrile/water from 15:85 to 60:40 in 70 min. Fractions containing homogeneous material as monitored by HPLC were combined and lyophilized; yield of colorless oil: 8 mg (10%); HPLC [column: Nucleosil 300/C8 (Macherey & Nagel, Düren Germany), linear gradient of acetonitrile/2% phosphoric acid from 5:95 to 80:20 in 30 min at a flow rate of 1 ml/min]: Rₜ = 7.99 min; ES-MS: *m/z =* 1464.6 ± (44.0)ₙ with n = 0 to 4 [M+H]⁺; calcd. Mᵣ = 1463.88 ± (44.03)ₙ with n = 0 to 4.

### (PEG)₁₈₋₂₅-[NH-CO-(CH₂)₂-CO-Leu-Leu-Nle-H]₂ (3)

(PEG)₁₈₋₂₅-[NH-CO-(CH₂)₂-CO-Leu-Leu-Nle-Sc]₂ (80 mg, 53 µmol) was dissolved in a mixture of 5 ml MeOH, 2 ml acetic acid and 2 ml formaldehyde (37 % in H₂0). After 2 h the solvent was removed and the oily residue was immediately purified by preparative HPLC on Nucleosil 250/C18 (Macherey & Nagel, Düren, Germany) with a linear gradient of acetonitrile/water from 15:85 to 60:40 in 70 min. Fractions containing homogeneous material as monitored by HPLC were combined and lyophilized; yield of colorless oil: 35 mg (20%); HPLC [column: Nucleosil 300/C8 (Macherey & Nagel, Düren, Germany), linear gradient of acetonitrile/2% phosphoric acid from 5:95 to 80:20 in 30 min at a flow rate of 1 ml/min]: Rₜ = 9.56 min; ES-MS: *m/z* = 1788.6 ± (44.0)ₙ with n = 0 to 4 [M+H]⁺; calcd. Mᵣ = 1787.14 ± (44.03)ₙ.

### References

1. Emori, Y., Tsukahara, T., Kawasaki, H., Ishiura, S., Sugita, H. and Suzuki, K. *Mol. Cell. Biol.* **11**, 344-353 (1991).
2. Rivett, A.J. *Biochem J*. **291**, 1-10 (1993); Peters, J.-M. *TIBS* **19**, 377-382 (1994); Goldberg, A.L., Stein, R. and Adams, J. *Chemistry & Biology* **2**, 503-508 (1995); Hochstrasser, M. *Curr. Opin. in Cell Biol.* **7**, 215-223 (1995); Rubin, D.M. and Finley, D. *Current Biology* **5**, 854-858 (1995); Lord, M. *Current Biology* **6***,* 1067-1069 (1996); Coux, O., Tanaka, K. and Goldberg, A.L. *Annu. Rev. Biochem.* **65**, 801-847 (1996).
2a. Stock, D., Nederlof, P.M., Seemüller, E., Baumeister, W., Huber, R. and Löwe, J. *Curr. Opin. Biotech.* **7***.* 376-385 (1996); Stock, D., Ditzel, L., Baumeister, W., Huber, R. and Löwe, J. *Cold Spring Harbor Symposia on Quantitative Biology* **LX**, 525-532 (1995).
3. Chen, P. and Hochstrasser, M. *EMBO J.* **14**, 2620-2630 (1995).
4. Groettrup, M., Soza, A., Kuckelkorn, U. and Kloetzel, P.M. *Immunol. Today* **17***,* 429-435 (1996).
5. Hilt, W. and Wolf, D.H. *TIBS* **21**, 96-102 (1996).
5a. Hilt, W., Enenkel, C., Gruhler, A., Singer, T. and Wolf, D.H. *J.Bio/.Chem.* **268**, 3479-3486 (1993).
6. Löwe, J., Stock, D., Jap, B., Zwickl, P., Baumeister, W. and Huber, R. *Science* **268**, 533-539 (1995).
7. Seemüller, E., Lupas, A., Stock, D., Löwe, J., Huber, R. and Baumeister, W. *Science* **268**, 579-582 (1995).
8. Brannigan, J. A., Dodson, G., Duggleby, H.J., Moody, P.C.E., Smith, J.L., Tomchick, D.R. and Murzin, A.G. *Nature* **378**, 416-419 (1995).
9. Heinemeyer, W., Tröndle, N., Albrecht, G.and Wolf, D.H. *Biochemistry* **33**,12229-12237 (1994).
10. Ehring, B., Meyer, T.H., Eckerskorn, C., Lottspeich, F. and Tampé, R. *Eur.J.Biochem.* **235**, 404-415 (1996).
11. Dick, L.R., Aldrich, C., Jameson, S.C., Moomaw, C.R., Pramanik, B.C., Doyle, C.K., DeMartino, G.N., Bevan, M.J., Forman, J.M. and Slaughter, C.A. *Immunol.* **152**, 3884-3894 (1994).
12. Wenzel, T., Eckerskorn, C., Lottspeich, F. and Baumeister, W. *FEBS Lett. 349,* 205-209 (1994).
13. Kuckelkorn, U., Frentzel, S., Kraft, R., Kostka, S., Groettrup, M. and Kloetzel, P.-M. *Eur.J.Immunol.* **25**, 2605-2611 (1995).
14. Groll, M., Ditzel, L., Löwe, J., Stock, D., Bochtler, M., Bartunik, H.D. and Huber, R. *Nature* **386**, 463-471 (1997).
15. Kopp, F., Kristensen, P., Hendil, K.B., Johnson, A., Sobek, A. and Dahlmann, B. *J. Mol. Biol.* **248**, 264-272 (1995).
16. Gaczynska, M., Rock, K.L. and Goldberg, A.L. *Nature* **365**, 264-267 (1993).
17. Driscoll, J., Brown, M.G., Finley, D. & Monaco, J.J. *Nature* **365**, 262-264 (1993).
18. Fehling, H.J., Swat, W., Laplace, C., Kühn, R., Rajewsky, K., Muller, U. and von Boehmer, H. *Science* **265**, 1234-1237 (1994).
19. Van Kaer, L., Ashton-Rickardt , P.G., Eichelberger, H., Gaczynska, M., Nagashima, K., Rock, K.L., Goldberg, A.L., Doherty, P.C. and Tonegawa, S. *Immunity* **1**, 533-541 (1994).
20. Adams, J. and Stein, R. *Annu. Reports in Med. Chem.* **31***,* 279-288 (1998).
21. Pereira, M.E., Nguyen, T., Wagner, B.J., Margolis, J.W., Yu, B., and Wilk, S. *J*. *Biol. Chem.* **267***,* 7949-7955 (1992).
22. Cardoza, C., Vinitsky, A., Hidalgo, M.C. and Orlowski, M. *Biochemistry* **31***,* 7373-7380 (1992).
23. Spaltenstein, A., Leban, J.J., Huang, J.J., Reinhardt, K.R., Viveros, O.H., Sigafoos and J., Crouch, R. *Tetrahedron Lett.* **37***,* 1343-1346 (1996).
24. Bogyo, M., McMaster, J.S., Gaczynska, M., Tortorella, D., Goldberg, A.L. and Ploegh, H. *Proc. Natl. Acad Sci. USA* **90***,* 6629-6634 (1997).
25. Bogyo, M., Shin, S., McMaster, J.S. and Ploegh, H. *Chemistry & Biology* **5***,* 307-320 (1998).
26. Reidlinger, J., Pike, A.M., Savory, P.J., Murray, R.Z. and Rivett, A.J.*J. Biol. Chem.* **272**, 24899-24095 (1997).
27. Fenteany, G., Standaert, R.F., Lane, W.S., Choi, S., Corey, E.J. and Schreiber, S.L. *Science* **268***,* 726-731 (1995).
28. Lynas, J.F., Harriot, P., Ilealy, A., McKervey, M.A. and Walker, B. *Bioorg. Med. Chem. Lett.* **8***,* 373-378 (1998).
29. Iqbal, M., Chatterjee, S., Kauer, J.C., Mallamo, J.P., Messina, P.A., Reiboldt, A. and Siman, R. *Bioorg. Med. Chem. Lett.* **6***,* 287-290 (1996).
30. Escherich, A., Ditzel, L.. Musiol, H.-J., Groll, M., Huber, R. and Moroder, L. *Biol. Chem.* **378***,* 893-898 (1997).
31. Loidl, G., Musiol, H.J., Groll, M., Ditzel, L., Huber, R. and Moroder, L. *25*^{th} *Eur. Peptide Symp.* Budapest, 30.8.-6.9.1998, Abstr.
32. Crothers, D.M. and Metzger, H., *Immunochemistry* **9**, 341-357 (1972).
33. Nen, D., Momo, M., Prospero, T. and Winter, G. *J Mol. Biol.* **246***,* 367-373 (1995).
34. Spike, C.G. and Parry, R.W. *J Am. Chem. Soc.* **75**, 2726-2729 (1953).
35. Schaschke, N., Musiol, H.-J., Assfalg-Machleidt, I., Machleidt, W., Rudolph-Böhner, S. and Moroder, L. *FEBS Lett.* **391**, 297-301 (1996).

## Claims

1. A bivalent proteasome inhibitor of the general formula I
X-CH(R1)-NH-[P]ₙ-Y-Z-Y'-[P']ₘ-NH-CH(R1')-X'
wherein X and X' are identical or different and are groups selected from aldehyde, boronic acid, boronic acid ester, ketoaldehyde, ketoester, ketoamide, chloromethylketone, trifluoromethylketone, diazomethane;
R1 and R1' are identical or different and correspond to the specificity of the S1 enzyme subsite for chymotrypsin- or trypsin-like or PGPH activity of the proteasome, selected from hydrophobic side chains of L- or D-natural and -unnatural amino acids, or amino-, guanido-, amidino-alkyl- or cycloalkyl-, alkaryl-, aralkyl residues and carboxy-alkyl- or aryl- or alkaryl- or aralkyl residues;
P and P' are identical or different and correspond to the P2 to P7 substrate positions and are natural or unnatural L- or D-amino acids with hydrophobic or hydrophylic or charged side chains;
m and n are identical or different and are 0, 1, 2, 3, 4, 5 or 6;
Y and Y' are identical or different and correspond to the formula CO-(A)-(B) where (A) is an alkyl, alkenyl, cycloalkyl, alkaryl, aralkyl or heteroaromatic group and (B) is CO or NH or omitted;
and Z is O-CH₂-CH₂-(O-CH₂-CH₂)ₙ-O, OC-CH₂-(O-CH₂-CH₂)ₙ₋₁-O-CH₂-CO or NH-CH₂-CH₂-(O-CH₂-CH₂)ₙ-NH with n varying from 4 to 50.

2. Inhibitor according to claim 1 wherein the said side chains are linear or branched alkyl, cycloalkyl, alkenyl, aryl, and -CH₂-R2 where R2 is aryl, aralkyl, alkaryl, cycloalkyl or -Q-R3 where Q is a chalcogen, and R3 is alkyl.

3. Inhibitor according to claim 1 or 2 wherein the alkyl and alkenyl groups per se or within aralkyl or alkaryl residues each contain from 1 to 20 resp. 2 to 20 C atoms.

4. Inhibitor according to claim 3 wherein the alkyl and alkenyl groups each contain 1 to 12 resp. 2 to 12 C atoms.

5. Inhibitor according to claim 4 wherein alkyl and alkenyl groups bound to an aryl residue contain 1 to 8 resp. 2 to 8 C atoms.

6. (Polyoxyethylene)₁₈₋₂₅-[NH-CO-(CH₂)₂-CO-Leu-Leu-Nle-H]₂.

7. Method for preparing a bivalent proteasome inhbitor characterized by linking monovalent proteasome inhibitor molecules with final amino or carboxy groups of a polyoxyethylene derivative containing 4 to 50 oxyethylene units.

8. Method according to claim 7 wherein a polyoxyethylene derivative containing 18 to 25 oxyethylene units is used.
